(19) Europäisches Patentamt
European Patent Office
Office européen des brevets

(11) **EP 3 424 475 A1**

(12) **EUROPEAN PATENT APPLICATION**
published in accordance with Art. 153(4) EPC

(43) Date of publication:
**09.01.2019  Bulletin 2019/02**

(51) Int Cl.:
**A61F 13/53** (2006.01)   **A61F 13/15** (2006.01)
**A61F 13/534** (2006.01)

(21) Application number: **17760190.3**

(22) Date of filing: **03.03.2017**

(86) International application number:
**PCT/JP2017/008613**

(87) International publication number:
**WO 2017/150728 (08.09.2017 Gazette 2017/36)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority:  **04.03.2016  JP 2016042671**

(71) Applicant: **Mitsui Chemicals, Inc.**
**Minato-ku**
**Tokyo 105-7122 (JP)**

(72) Inventors:
• **SAITA, Shohei**
  **Sodegaura-shi**
  **Chiba 299-0265 (JP)**
• **MATSUBARA, Akio**
  **Sodegaura-shi**
  **Chiba 299-0265 (JP)**
• **SUZUKI, Kenichi**
  **Tokyo 105-7122 (JP)**

(74) Representative: **Hoffmann Eitle**
**Patent- und Rechtsanwälte PartmbB**
**Arabellastraße 30**
**81925 München (DE)**

(54) **ABSORBENT BODY AND SANITARY ARTICLE**

(57)    An absorber having an absorption layer including a superabsorbent polymer and a spunbond nonwoven fabric composed of an olefinic polymer composition.

FIG. 5

**Description**

Technical Field

The present invention relates to an absorber and a sanitary article.

Background Art

Sanitary articles, such as a diaper and a sanitary napkin, generally include an absorption layer containing an absorber for absorbing a body fluid, such as urine or menstrual blood, between a back sheet constituted with a liquid-tight resin sheet (liquid-impermeable sheet) and a top sheet (liquid-permeable sheet) to be used inside in direct contact with the wearer's skin. As an absorber used in sanitary articles, such as a diaper and a sanitary napkin, a superabsorbent polymer (SAP) is often used.

Since SAP has higher absorbency than pulp, the absorbency of body fluid is superior to an article in which the absorption layer is composed solely of pulp, and a SAP absorption layer can be made thinner. In addition, SAP is in a powder form in a dry state, and swells to solidify into a gel form, when it absorbs a body fluid, so that the absorbed body fluid may be retained securely in the absorption layer, and backflow or leakage of the absorbed body fluid may be prevented.

In recent years, the demand for thinner sanitary articles is increasing from the viewpoints of designability, convenience of carrying it, high efficiency at the time of distribution, and the like. In particular, for adult-use diapers, this demand is emerging obvious from the sense of resistance to wearing diapers. Furthermore, in order to reduce the environmental burden, demand for so-called environmentally friendly products able to reduce waste in disposing diapers, and avoid to the extent possible the use of natural materials that require a long period of time to grow as in the case of trees, is also increasing, and there is a growing tendency to reduce the use of pulp originated from wood resources.

To reduce the thickness of the diaper currently distributed commercially, it has been necessary to reduce the amount of an absorber, which occupies 50 to 70%, or even more, of the total mass of the diaper, especially the amount of bulky fluff pulp, which occupies 40% or more of the total mass of the absorber. Therefore, it has been believed as a practical improving method for reducing the thickness, that the amount of fluff pulp is reduced, while the absorption capacity is secured to satisfy the designed requirements for the absorptive article. However, the fluff pulp has an important role of fixing SAP, and letting the liquid flow through the absorber. Therefore, in a case in which the amount of the fluff pulp is less than 40% of the total mass of the absorber, the fixation of SAP becomes insufficient, and adjacent SAPs stick together when a liquid is absorbed to cause a trouble of disturbance of liquid flow (gel blocking). When gel blocking occurs, backflow of a body fluid from the absorber takes place, which may give discomfort to the diaper wearer.

Therefore, various methods have been proposed to realize reduction of the thickness, while suppressing occurrence of gel blocking.

For example, a thin absorber in which three layers of a diffusion layer, an absorption layer, and a base material layer are laminated in this order and integrated by thermal bonding, wherein the diffusion layer is composed of fibers and has a function of diffusing a body fluid, the absorption layer for absorbing a body fluid is constituted with an air-laid web containing a uniform mixture of a superabsorbent polymer and a synthetic fiber, and the base material layer prevents shedding of the superabsorbent polymer from the absorption layer, characterized in that the fiber length of the synthetic fiber in the absorption layer is from 2 to 6 mm, and the synthetic fiber is randomly dispersed in the air-laid web forming a three-dimensional network structure to retain the superabsorbent polymer, and that the thickness of the absorber is from 0.5 to 4 mm, and the basis weight of the absorber is from 50 to 300 g/m$^2$, has been proposed (see Japanese Patent Application Laid-Open (JP-A) No. 2005-095481).

SUMMARY OF INVENTION

Problems to be Solved by the Invention

However, with respect to the technique according to JP-A No. 2005-095481, the producing process for obtaining a desired layer configuration is complicated, and for obtaining practically acceptable interlayer adhesiveness, it is necessary to raise the temperature and pressure of a calendar roll. Therefore, by the technique according to JP-A No. 2005-095481, the space having existed inside the absorber is diminished, and as a result the distance between the absorbers is shortened and gel blocking becomes prone to occur. When gel blocking occurs, backflow of a body fluid once absorbed may take place.

The present invention was made in view of the above problems, to achieve the following object.

In other words, an object of the invention is to provide an absorber, which is produced by a simple producing process without using an adhesive, contains a not unevenly distributed superabsorbent polymers (SAP), and is superior in body fluid absorption performance (for example, diffusing capacity, or absorption rate), and in which backflow of a body fluid once absorbed is suppressed, and a sanitary article having the absorber.

Solution to Problem

The means for achieving the object includes the following modes.
<1> An absorber having an absorption layer including a superabsorbent polymer and a spunbond nonwoven fabric composed of an olefinic polymer composition.
<2> The absorber according to <1>, wherein the olefinic polymer composition includes a hydrophilizing agent.
<3> The absorber according to <1> or <2>, wherein the spunbond nonwoven fabric is a crimped spunbond nonwoven fabric.
<4> The absorber according to any one of <1> to <3>, wherein the absorption layer is an embossed article.
<5> The absorber according to any one of <1> to<4>, wherein the absorption layer further includes a melt-blown nonwoven fabric.
<6> The absorber according to any one of <1> to <5>, wherein the absorption layer further includes synthetic pulp or natural fluff pulp.
<7> The absorber according to <6>, wherein the synthetic pulp or the natural fluff pulp is present on a surface of the absorption layer.
<8> The absorber according to <7>, wherein from 0.01 to 10 parts by mass of polypropylene glycol having a molecular weight of from 200 to 10000 is adhered to 100 parts by mass of the synthetic pulp.
<9> The absorber according to any one of <1> to <8> including further an elastic nonwoven fabric.
<10> A sanitary article including the absorber according to <9>.
<11> A method of producing an absorber having an absorption layer including a superabsorbent polymer and a spunbond nonwoven fabric composed of an olefinic polymer composition, the method including:

step (I): forming long fibers from a melted olefinic polymer composition, drawing to thin the long fibers, and accumulating the long fibers on a collecting belt to obtain a spunbond nonwoven fabric,
step (II): sprinkling a superabsorbent polymer onto the spunbond nonwoven fabric on the collecting belt,
step (III): obtaining a laminate by layering, on the collecting belt, a spunbond nonwoven fabric different from the spunbond nonwoven fabric on the collecting belt, and
step (IV): performing a surface shaping processing on the laminate on the collecting belt.

<12> The method of producing an absorber according to <11>, wherein the olefinic polymer composition includes a hydrophilizing agent.
<13> The method of producing an absorber according to <11>, wherein the spunbond nonwoven fabric composed of the olefinic polymer composition is a crimped spunbond nonwoven fabric.
<14> The method of producing an absorber according to <11>, wherein in the step (II) the superabsorbent polymer is sprinkled on the spunbond nonwoven fabric composed of the olefinic polymer composition intercalating another layer.
<15> The method of producing an absorber according to <11>, wherein in the step (II) synthetic pulp or natural fluff pulp is further sprinkled on the spunbond nonwoven fabric composed of the olefinic polymer composition.

Advantageous Effects of Invention

According to the invention, an absorber, which is produced by a simple producing process without using an adhesive, contains a not unevenly distributed superabsorbent polymers (SAP), and is superior in body fluid absorption performance (for example, diffusing capacity, and absorption rate), and in which backflow of a body fluid once absorbed is suppressed, and a sanitary article having the absorber are provided.

BRIEF DESCRIPTION OF DRAWINGS

[FIG. 1A] FIG. 1A is a diagram schematically showing a cross section of a long fiber of a spunbond nonwoven fabric included in an absorber according to an example of the invention. In the figure, the blank region represents a resin.
[FIG. 1B] FIG. 1B is a diagram schematically showing a cross section of a long fiber of a spunbond nonwoven fabric included in an absorber according to an example of the invention. In the figure, the blank region and the dotted region represent respective resins to be combined.

[FIG. 2] FIG. 2 is a schematic diagram showing an example of a producing apparatus of a spunbond nonwoven fabric for producing an absorber according to an example of the invention.

[FIG. 3] FIG. 3 is an optical micrograph of a superabsorbent polymer used in an Example of the invention.

[FIG. 4] FIG. 4 is a view schematically showing an embossed pattern of an embossing roll used in an Example of the invention.

[FIG. 5] FIG. 5 is a schematic diagram showing an in-line process for producing the absorber according to the invention.

DESCRIPTION OF EMBODIMENTS

A numerical range expressed by "x to y" includes herein the values of x and y in the range as the minimum and maximum values, respectively.

[Absorber]

An absorber according to the invention has an absorption layer including a superabsorbent polymer and a spunbond nonwoven fabric composed of an olefinic polymer composition.

With respect to an absorber according to the invention, production by a simple process without using an adhesive is possible, and uneven distribution of SAP, namely a superabsorbent polymer resin, may be suppressed by spunbond fibers (preferably embossed).

This realizes superior absorption performance (diffusing capacity, and absorption rate) and suppression of backflow of a body fluid once absorbed. Therefore, it becomes possible to reduce the thickness of the absorber.

Particulars of each component, *etc.* constituting an absorber according to the invention will be described below.

<Absorption Layer>

The absorption layer of an absorber according to the invention contains a superabsorbent polymer.

In addition, the absorption layer of an absorber according to the invention includes a spunbond nonwoven fabric composed of an olefinic polymer composition.

The absorption layer of an absorber according to the invention is preferably an embossed article.

(Superabsorbent Polymer)

As a superabsorbent polymer, for example, a commercially available superabsorbent polymer may be used.

Examples of the superabsorbent polymer include a hydrolyzate of a starch/acrylonitrile graft copolymer, a neutralized product of a starch/acrylic acid graft polymer, a saponified product of a vinyl acetate/acrylate copolymer, and a partially neutralized poly(acrylic acid). Among these superabsorbent polymers, a partially neutralized poly(acrylic acid) is preferable from the viewpoints of production amount, production cost, water absorption performance and the like.

Examples of a method of synthesizing a partially neutralized poly(acrylic acid) include a reverse phase suspension polymerization method, and an aqueous solution polymerization method.

As the partially neutralized poly(acrylic acid), powdered or granular ones are on the market.

In this regard, a superabsorbent polymer means a polymer capable of absorbing pure water more than 300 times as much as its own weight.

The average particle diameter of a superabsorbent polymer is preferably from 100 to 600 $\mu$m, more preferably from 150 to 550 $\mu$m, and further preferably from 200 to 500 $\mu$m from the viewpoint of prevention of dissipation of the superabsorbent polymer in the absorber and gel blocking phenomenon on the occasion of water absorption, and also improvement of the texture by reducing a rough feel of the absorber. When an absorbent resin with a size less than 100 $\mu$m is used, it becomes difficult for spunbond fibers to retain the absorbent resin, and therefore there arises a risk that gel blocking phenomenon may occur on the occasion of water absorption due to movement of the absorbent resin.

The amount of the superabsorbent polymer in an absorber according to the invention may be appropriately selected according to the design of the absorber, and it is preferably about from 15 to 70 parts by mass with respect to 100 parts by mass of the absorber, and more preferably from 30 to 70 parts by mass.

(Spunbond Nonwoven Fabric)

The absorption layer of an absorber according to the invention includes a spunbond nonwoven fabric.

A spunbond nonwoven fabric included in the absorption layer of an absorber according to the invention is composed of an olefinic polymer composition.

(Olefinic Polymer Composition)

The olefinic polymer composition constituting a spunbond nonwoven fabric contains an olefinic polymer.

The olefinic polymer is, for example, a homopolymer or a copolymer of an $\alpha$-olefin, such as ethylene, propylene, 1-butene, 1-hexene, 4-methyl-1-pentene, and 1-octene.

Specific examples of the olefinic polymer include a crystalline olefinic polymer, and the polymer includes an ethylene polymer, such as high pressure low density polyethylene, linear low density polyethylene (*i.e.* LLDPE: ethylene/$\alpha$-olefin random copolymer), or high density polyethylene; a propylene polymer, such as a propylene homopolymer, or a propylene/a-olefin copolymer; a 1-butene polymer, such as a 1-butene homopolymer or a 1-butene/$\alpha$-olefin copolymer; and a 4-methyl-1-pentene polymer, such as a 4-methyl-1-pentene homopolymer, or a 4-methyl-1-pentene/$\alpha$-olefin copolymer.

As the propylene polymer, use of a propylene/a-olefin random copolymer having a melting point (Tm) in a range of from 125 to 155°C (preferably from 130 to 147C) that is a copolymer of propylene and a small amount of one, or two or more kinds of $\alpha$-olefins having 2 or more (preferably 2 to 8) carbon atoms, such as ethylene, 1-butene, 1-pentene, 1-hexene, 1-octene, or 4-methyl-1-pentene, is especially preferable, because a nonwoven fabric superior in flexibility and initial hydrophilicity can be obtained.

Although there is no particular restriction on the content of an $\alpha$-olefin in a propylene/a-olefin random copolymer, it is, for example, from 1 to 10 mol%, and more preferably from 1 to 5 mol%.

The MFR (melt flow rate) of the propylene polymer is, for example, from 15 to 800 g/10 min, preferably from 10 to 100 g/10 min, and more preferably from 30 to 80 g/10 min. A MFR is measured based on the standard with respect to each resin. For example, the MFR of a propylene polymer is measured at 230°C under a load of 2.16 kg based on ASTM D1238.

The ratio Mw/Mn of the weight average molecular weight (Mw) to the number average molecular weight (Mn) of the propylene polymer is, for example, from 1.5 to 5.0. The Mw/Mn of the propylene polymer is preferably from 1.5 to 3.0 from the viewpoint of obtaining a conjugate fiber having good spinnability and particularly excellent fiber strength.

Good spinnability means herein a character that no filament breakage nor fusion of filaments occurs at the time of extrusion from a spinning nozzle or at the time of drawing.

A Mw/Mn is herein a value measured by a usual method using GPC (gel permeation chromatography).

An olefinic polymer composition may contain, if necessary, another component to the extent that the object of the invention is not impaired.

As another component, an additive which is capable of imparting another function may be selected according to an application and added appropriately to the extent that the object of the invention is achieved.

Examples of other components include a stabilizer (more particularly, various stabilizers, such as a heat stabilizer, or a weathering stabilizer), a filler, an antistatic agent, a slip agent, an anti-blocking agent, an anti-fogging agents, a lubricant, a dye, a pigment, a natural oil, a synthetic oil, and a wax.

Examples of the stabilizer include an age resister, such as 2,6-di-*t*-butyl-4-methylphenol (BHT); a phenolic antioxidant, such as tetrakis[methylene-3-(3,5-di-*t*-butyl-4-hydroxyphenyl)propionate]methane, $\beta$-(3,5-di-*t*-butyl-4-hydroxyphenyl)propionic acid alkyl ester, 2,2'-oxazamide bis[ethyl-3-(3,5-di-*t*-butyl-4-hydroxyphenyl)]propionate, Irganox 1010 (trade name, hindered phenol based antioxidant); a fatty acid metal salt, such as zinc stearate, calcium stearate, and calcium 1,2-hydroxystearate; and a polyhydric alcohol fatty acid ester, such as glycerin monostearate, glycerin distearate, pentaerythritol monostearate, pentaerythritol distearate, and pentaerythritol tristearate. These may be used in combination.

Examples of the filler include silica, diatomaceous earth, alumina, titanium oxide, magnesium oxide, pumice powder, pumice balloon, aluminum hydroxide, magnesium hydroxide, basic magnesium carbonate, dolomite, calcium sulfate, potassium titanate, barium sulfate, calcium sulfite, talc, clay, mica, asbestos, calcium silicate, montmorillonite, bentonite, graphite, aluminum powder, and molybdenum sulfide.

(Hydrophilizing Agent)

The olefinic polymer composition preferably contains a hydrophilizing agent.

When the olefinic polymer composition contains a hydrophilizing agent, the liquid spreading property of a spunbond nonwoven fabric composed of an olefinic polymer composition is improved, and liquid absorption utilizing a superabsorbent polymer in broader area of the in-plane direction of the absorption layer becomes possible, so that an absorber having adequate absorbency may be obtained, even when the content of the superabsorbent polymer in the absorption layer is low. Furthermore, it is preferable because it becomes possible to reduce the content of a superabsorbent polymer in an absorber, to suppress gel blocking, and to mitigate rewetting after water absorption.

As the hydrophilization treatment means for adding a hydrophilizing agent into an olefinic polymer composition (spunbond nonwoven fabric), for example, the following known methods may be applied singly or in combination.

Examples thereof include a method by which a nonwoven fabric (spunbond nonwoven fabric) is produced by a spunbonding method using a raw material prepared by adding a hydrophilizing agent to a hydrophobic artificial fiber; a method by which a hydrophilizing agent is entrained in forming a spunbond nonwoven fabric with a hydrophobic artificial fiber; and a method by which a hydrophilizing agent is impregnated into a spunbond nonwoven fabric after preparation with a hydrophobic artificial fiber.

Examples of the hydrophilizing agent include an anionic surfactant, such as an aliphatic sulfonate, and a higher alcohol sulfate salt; a cationic surfactant, such as a quaternary ammonium salt; a nonionic surfactant, such as a polyethylene glycol fatty acid ester, a polyglycerin fatty acid ester, a sorbitan fatty acid ester, and a di-saturated fatty acid ester of polyalkylene glycol; a silicone surfactant, such as a polyoxyalkylene-modified silicone; and a stain release agent composed of a resin based on a polyester, a polyamide, an acrylic resin, or a urethane resin.

As the hydrophilizing agent, a di-saturated fatty acid ester of polyalkylene glycol is particularly preferable. In a case in which a nonwoven fabric (spunbond nonwoven fabric) is produced by a spunbonding method using a raw material prepared by adding a hydrophilizing agent to a hydrophobic artificial fiber, the content of the hydrophilizing agent is preferably from 1 to 5 parts by mass with respect to 100 parts by mass of the raw material.

As the di-saturated fatty acid ester of polyalkylene glycol is preferably a di-saturated fatty acid ester of a polyalkylene glycol represented by the following Formula (1).

$$R\text{-}COO\text{-}(C_nH_{2n}O)_x\text{-}CO\text{-}R \qquad \text{Formula (1)}$$

[wherein, R is an alkyl group having from 13 to 17 carbon atoms, n is an integer from 2 to 4, and x is an integer from 10 to 23.]

Specifically, R in Formula (1) is a $CH_3\text{-}(CH_2)_{12}$ group (tridecyl group), a $CH_3\text{-}(CH_2)_{13}$ group (tetradecyl group), a $CH_3\text{-}(CH_2)_{14}$ group (pentadecyl group), a $CH_3\text{-}(CH_2)_{15}$ group (hexadecyl group), a $CH_3\text{-}(CH_2)_{16}$ group (heptadecyl group), or a $CH_3\text{-}(CH_2)_{17}$ group (octadecyl group).

In consideration of the initial hydrophilicity, R is preferably a $CH_3\text{-}(CH_2)_{12}$ group (tridecyl group), a $CH_3\text{-}(CH_2)_{13}$ group (tetradecyl group), a $CH_3\text{-}(CH_2)_{14}$ group (pentadecyl group), or a $CH_3\text{-}(CH_2)_{15}$ group (hexadecyl group).

In Formula (1), the $(C_nH_{2n}O)_x$ group is specifically a polyethyleneoxy group, a polypropyleneoxy group, or a polybutyleneoxy group. The average molecular weight of the $(C_nH_{2n}O)_x$ group is preferably from 400 to 1000, and more preferably from 600 to 800.

A spunbond nonwoven fabric containing the hydrophilizing agent exhibits a liquid strike-through time of, for example, 7 sec or less, preferably 5 sec or less, and more preferably 3 sec or less, when the evaluation according to EDANA 150, 2-93 is repeated three times.

In addition, the water retention rate under no load is, for example, 200% or more, preferably 300% or more, and more preferably 400% or more. The total basis weight of a spunbond nonwoven fabric included in an absorber is usually from 10 to 300 $g/m^2$, preferably from 10 to 200 $g/m^2$, and more preferably from 10 to 150 $g/m^2$. When it is thicker than 300 $g/m^2$, thickness reduction may be inadequate. On the other hand, when it is thinner than 10 $g/m^2$, there is a risk that the nonwoven fabric constituting the absorber may be broken when SAP absorbs a body fluid and swells.

(Crimped Spunbond Nonwoven Fabric)

The spunbond nonwoven fabric may be a crimped spunbond nonwoven fabric.

When a crimped spunbond nonwoven fabric is used as the spunbond nonwoven fabric, it is bulkier compared to a case where a spunbond nonwoven fabric composed of non-crimped fibers is used. Therefore, a mode using a crimped spunbond nonwoven fabric as the spunbond nonwoven fabric is advantageous in that a sufficient porosity can be secured even in a state with a load when the wearer is sitting or lying down, and also in that gel blocking is suppressed and the liquid absorption rate is not impaired even when the basis weight is low.

A fiber for a crimped spunbond nonwoven fabric is a fiber prepared by melt-spinning a thermoplastic resin, and then crimping the same by cooling, which is preferably an actually crimped long fiber having usually a number of crimps of 5/25 mm or more (preferably 20/25 mm or more, and more preferably 25/25 mm or more).

The fiber of a crimped spunbond nonwoven fabric has a fiber diameter of, for example, from 0.5 to 5.0 d, preferably from 0.5 to 3.0 d, and more preferably from 0.5 to 2.0 d.

As an example of a crimped spunbond nonwoven fabric, there is an actually crimped spunbond nonwoven fabric, for which a fiber has been crimped due to strains generated by cooling. Examples of a long fiber of an actually crimped spunbond nonwoven fabric include an eccentric sheath-core conjugate long fiber, a parallel (side by side type) conjugate long fiber, and an eccentric hollow conjugate long fiber conjugating different kinds of thermoplastic resins different in crystallization temperature, melting point, softening point, crystallization speed, melt viscosity, *etc.*

- Parallel Crimped Spunbond Nonwoven Fabric -

A spunbond nonwoven fabric may be also a parallel crimped spunbond nonwoven fabric.

One of preferred modes of a parallel crimped spunbond nonwoven fabric is a parallel crimped spunbond nonwoven fabric which is composed of at least two thermoplastic resins having a melting point difference of 5C or more, and in which the ratio of the part of the high melting point thermoplastic resin (A) to the part of the low melting point thermoplastic resin (B) [the part of the high melting point thermoplastic resin (A) / the part of the low melting point thermoplastic resin (B)] is from 5/95 to 95/5 (mass ratio) (more preferably from 5/95 to 50/50 (mass ratio), and further preferably from 5/95 to 30/70 (mass ratio)).

One of preferred modes of a parallel crimped spunbond nonwoven fabric different from the above is a spunbond nonwoven fabric of crimped conjugate fibers composed of a first propylene polymer component and a second propylene polymer component, wherein the first and second propylene polymer components are a propylene homopolymer or a propylene/ethylene random copolymer having respectively an ethylene unit content of from 0 to 10 mol%, and a melt flow rate measured according to ASTM D 1238 (MFR: measurement temperature of 230°C, and load of 2.16 kg) of from 20 to 200 g/10 min, wherein the MFR ratio (First propylene polymer component / Second propylene polymer component) between the first propylene polymer component and the second propylene polymer component is from 0.8 to 1.2, wherein the first and second propylene polymer components are arranged such that the two occupy substantially separate zones in the cross section of the crimped conjugate fiber and extend continuously along the length direction, while the second propylene polymer component constitutes at least part of the circumferential surface extending continuously along the length direction of the crimped conjugate fiber, wherein the melting point measured by a differential scanning calorimeter (DSC) of the first propylene polymer component is higher than that of the second propylene polymer component by 20C or more, and wherein the component ratio in terms of the mass ratio of the first propylene polymer component / the second propylene polymer component is from 20/80 to 5/95, and as a consequence an eccentric parallel crimped spunbond nonwoven fabric constituted with a core made of the first propylene polymer component, and a sheath made of the second propylene polymer component is formed.

Further, as the parallel crimped spunbond nonwoven fabric, for example, a nonwoven fabric according to WO 2010/050407 or WO 2012/129211 may be used.

(Melt-blown Nonwoven Fabric)

An absorption layer of the absorber according to the invention may contain a melt-blown nonwoven fabric.

A melt-blown nonwoven fabric may be produced by a known method.

For example, as a method of forming directly a melt-blown nonwoven fabric on a spunbond nonwoven fabric, a melt blown method, by which a melted thermoplastic resin is sprayed onto the surface of the spunbond nonwoven fabric to accumulate fibers thereon, may be performed. In doing so, it is preferable that the side of the spunbond nonwoven fabric opposite to the side on which a melted material is to be sprayed is evacuated to a negative pressure, and fibers prepared by a melt-blown method are sprayed to accumulate thereon, and at the same time the spunbond nonwoven fabric and the melt-blown nonwoven fabric are united (laminated) to obtain a nonwoven fabric laminate.

As a melt-blown nonwoven fabric, a melt-blown nonwoven fabric composed of polyolefin fibers, in which (I) the average fiber diameter is 2 $\mu$m or less, (ii) the CV value of the fiber diameter distribution is 60% or less (preferably 50% or less), and (iii) the number of fused nodes per 100 fibers is 15 or less (preferably 12 or less, and more preferably 10 or less), is preferable.

A melt-blown nonwoven fabric having the above characteristics can be produced, for example, by the method described in WO2012/077638.

When an absorption layer of the absorber according to the invention includes a melt-blown nonwoven fabric, the melt-blown nonwoven fabric may include a hydrophilizing agent as well as the spunbond nonwoven fabric. As the hydrophilizing agent, the aforementioned hydrophilizing agent may be used.

A nonwoven fabric laminate constituted with a spunbond nonwoven fabric containing the hydrophilizing agent and a melt-blown nonwoven fabric containing the hydrophilizing agent exhibits a liquid strike-through time of, for example, 30 sec or less, preferably 20 sec or less, and more preferably 10 sec or less, when the evaluation according to EDANA 150, 2-93 is repeated three times.

Further, the nonwoven fabric laminate has a water retention rate with no load of, for example, 300% or more, preferably 500% or more, and more preferably 700% or more.

When the nonwoven fabric laminate is allowed to stand on a slope of 45° and 80 mL of physiological saline is dropped, the flow distance of the liquid is, for example, 400 mm or less, preferably 300 mm or less, and more preferably 200 mm or less.

The total basis weight of the melt blown layer included in the absorber is usually from 0.5 to 50 g/m$^2$, preferably from 0.5 to 25 g/m$^2$, and more preferably from 0.5 to 10 g/m$^2$. When it is thicker than 50 g/m$^2$, since the melt blown layer retains the liquid, the liquid permeability to SAP tends to deteriorate and the wearer may feel uncomfortable.

(Synthetic Pulp and Natural Fluff Pulp)

An absorption layer of the absorber according to the invention may contain synthetic pulp or natural fluff pulp to the extent that the advantageous effect of the invention is not impaired.
In a case in which the absorption layer contains synthetic pulp or natural fluff pulp, the retention of SAP is enhanced, and gel blocking may be easily suppressed, even when the thickness of the absorber is reduced.

(Synthetic Pulp)

The material composing synthetic pulp is preferably a polyolefin resin.
Preferable examples of the polyolefin resin that may be contained in the synthetic pulp include an ethylene homopolymer, an ethylene/$\alpha$-olefin copolymer, a propylene homopolymer, a propylene/$\alpha$-olefin copolymer, an ethylene/vinyl acetate copolymer, an ethylene/(meth)acrylic acid copolymer, an ethylene/(meth)acrylic acid ester copolymer, a maleated polyethylene, a maleated polypropylene, and a blended resin thereof.
In this regard, (meth)acrylic acid means acrylic acid, or methacrylic acid.
An ethylene homopolymer which may be used for synthetic pulp has a melt flow rate (MFR, ASTM D 1238, 190°C, load of 2.16 kg) of, for example, from 0.01 to 1000 g/10 min, preferably from 0.05 to 500 g/10 min, and more preferably from 0.1 to 100 g/10 min.
When an ethylene homopolymer having a MFR within the above range is used, a synthetic pulp, in which fibers are highly fibrillated, and entangled with each other, and which is superior in powder retention can be obtained.
Meanwhile, a propylene homopolymer which can be used for synthetic pulp has a melt flow rate (MFR, ASTM D 1238, 230°C, load of 2.16 kg) of, for example, from 0.1 to 500 g/10 min, preferably from 0.5 to 100 g/10 min, and more preferably from 1 to 50 g/10 min.
When a propylene homopolymer having a MFR within the above range is used, a synthetic pulp, in which fibers are highly fibrillated, and entangled with each other, and which is superior in powder retention may be obtained.
A known method may be applied for producing synthetic pulp from a polyolefin resin. A known method is described in detail, for example, in Encyclopedia of Chemical Technology, 3rd ed., Vol. 19, p. 420-425.
Examples of a known method include a method, by which melt-spun fibers are cut short and refined, and a method, by which melt-flashed or emulsion-flashed fibers are refined.
When the absorber according to the invention includes synthetic pulp, the synthetic pulp may be hydrophilized.
As a mode in which the synthetic pulp is hydrophilized, a mode in which from 0.01 to 10 parts by mass of polypropylene glycol having a molecular weight of from 200 to 10,000 is adhered to 100 parts by mass of the synthetic pulp is preferable.
As polypropylene glycol to be adhered to the synthetic pulp is preferably a polypropylene glycol having a molecular weight of from 200 to 10000 (more preferably from 400 to 6000) obtained by polymerizing propylene oxide by a conventional method.
In this regard, the molecular weight of a polypropylene glycol is a value determined by gel permeation chromatography (GPC) and is calculated by a comparison of the OH numbers between a reference PPG with a known OH number and a sample PPG.
When the molecular weight of a polypropylene glycol is 200 or more, adhesion to synthetic pulp is improved. When the molecular weight of a polypropylene glycol is 10000 or less, the effect of imparting hydrophilicity is improved.
The amount of the polypropylene glycol adhered to synthetic pulp is preferably from 0.01 to 10 parts by mass, and particularly preferably from 0.05 to 5 parts by mass with respect to 100 parts by mass of the synthetic pulp.
When the amount of the polypropylene glycol adhered to 100 parts by mass synthetic pulp is 0.01 part by mass or more, the effect of improving the hydrophilicity of the synthetic pulp is obtained, and when it is 10 parts by mass or less, deterioration of the performance of the synthetic pulp may be suppressed.
Examples of a method of adhering polypropylene glycol to synthetic pulp include a method by which a polypropylene glycol, or an aqueous solution or an aqueous dispersion thereof is sprayed on synthetic pulp; a method by which a polypropylene glycol, or an aqueous solution or an aqueous dispersion thereof is added to an aqueous slurry of synthetic pulp and mixed; and a method by which synthetic pulp is added to an aqueous solution or an aqueous dispersion of a polypropylene glycol and mixed.
While a polypropylene glycol is mixed with stirring in an aqueous solution or an aqueous dispersion, it may be adsorbed on the surface of synthetic pulp and may adhere thereto.
The temperature in adhering a polypropylene glycol to synthetic pulp is preferably from 0 to 90°C, and room temperature is convenient.
The average fiber length of a synthetic pulp and a natural fluff pulp, which may be used according to the invention is preferably from 0.05 mm to 10 mm, and more preferably from 0.1 mm to 6 mm from the viewpoint of suppressing of movement of a superabsorbent polymer.

The amount of the synthetic pulp or the natural fluff pulp in the absorption layer according to the invention is preferably from 1% by mass to 20% by mass, more preferably from 1% by mass to 10% by mass, and further preferably from 1% by mass to 5% by mass with respect to the amount of absorption layer from the viewpoint of suppresses of movement of a superabsorbent polymer without imparing the performance of the absorption layer.

A commercially available product may be used as a synthetic pulp that may be contained in the absorption layer.

Examples of a commercially available synthetic pulp include Synthetic Pulp [SWP (registered trademark), produced by Mitsui Chemicals, Inc.].

(Embossed Article)

An absorption layer of the absorber according to the invention is preferably an embossed article.

A mode in which the absorption layer is an embossed article is preferable, because the production process is simple and an absorber having a porosity suitable for retaining SAP can be produced easily.

The embossed area in the embossed article is, for example, from 3 to 30%, preferably from 3 to 20%, and more preferably from 3 to 12%.

Examples of the embossed pattern of an embossed part include various known shapes, such as a round shape, an elliptic shape, a triangular shape, a quadrangular shape (for example, a rhombic shape), a polygonal shape having five-sides or more. As the embossed pattern of an embossed part, a demarcated shape is preferable for retaining SAP evenly.

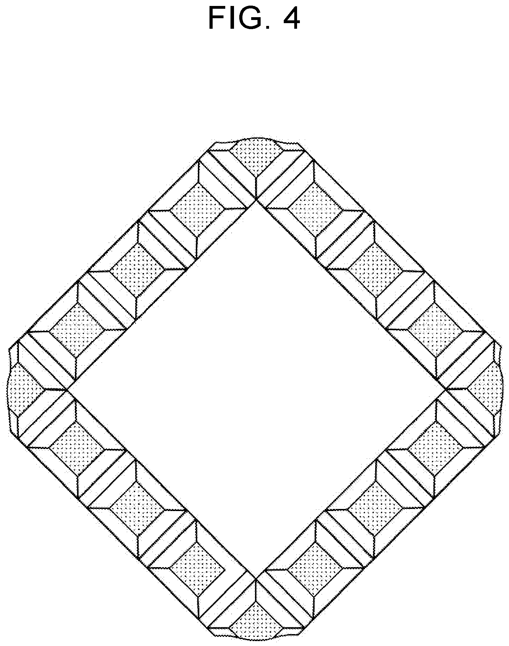

For example, the quilt pattern shown in FIG. 4 is preferable, because the degree of freedom of the fiber is high with respect to a SAP swollen after absorbing a liquid, and consequently the absorber hardly breaks.

Further, as described in WO2011/122277, an article is configured with embossed patterns having a unit pattern demarcated by an emboss line, such that a plurality of embossed elements are arranged continuously but with certain gaps defined by the emboss line, and that at least one of the plurality of embossed elements is placed in an outward direction along the shortest path from an optional position in a non-embossed area in the unit pattern to the emboss line demarcating the non-embossed area so as to block the outward direction extending the shortest path from the optional position beyond the emboss line demarcating the non-embossed area.

There is no particular restriction on a method of producing the absorption layer which is an embossed article.

Examples of a method of producing an absorption layer, which is an embossed article, include a method, by which, for example, using a known spunbond nonwoven fabric producing apparatus, a melted thermoplastic resin is extruded through a spinneret for non-crimped fibers, or a spinneret for crimped fibers to form long fibers, the long fibers are cooled and drawn to thin to obtain a predetermined fineness by cooling air, and the long fibers with a predetermined fineness are collected on a collecting belt to accumulate up to a predetermined thickness (basis weight) thereby obtaining a spunbond nonwoven fabric; then SAP is sprinkled evenly on the spunbond nonwoven fabric using a known absorber sprinkler device; then another spunbond nonwoven fabric having a predetermined thickness is layered on the SAP sprinkled spunbond nonwoven fabric to form a laminate; and then embossing is performed on the laminate with an embossing roll.

Further, before or after sprinkling SAP on the spunbond nonwoven fabric, a melt-blown nonwoven fabric may be laminated with the spunbond nonwoven fabric using a known melt-blown nonwoven fabric producing apparatus.

Further, synthetic pulp or natural fluff pulp may be contained in the absorption layer. Examples of a method of producing an absorption layer containing synthetic pulp or natural fluff pulp include a method by which fluffed synthetic pulp or natural fluff pulp is shaped into a sheet form by a known method and then laminated with a spunbond nonwoven fabric, and a method by which fluffed synthetic pulp or natural fluff pulp is mixed with a fibrous superabsorbent polymer in a blender, *etc.,* and then the mixture is sprinkled evenly on a spunbond nonwoven fabric.

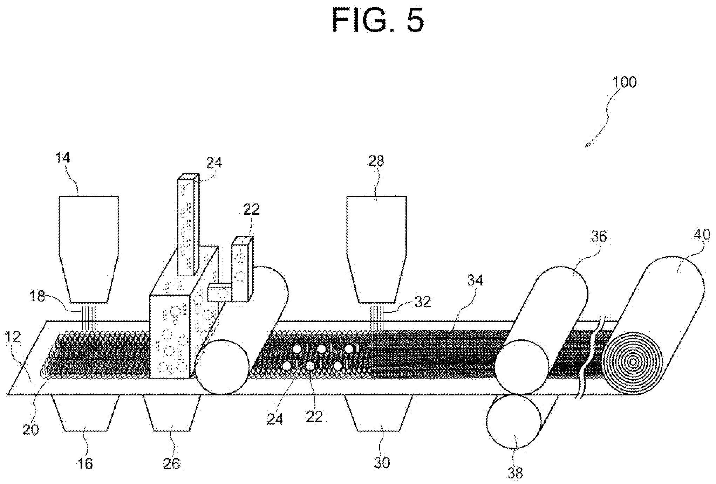

As a method of producing an absorber according to the invention having the absorption layer which is an embossed article, an in-line producing method as shown in FIG. 5 is preferable. The method of producing an absorber according to the invention by the producing apparatus 100 shown in FIG. 5 may be a method including a step (I) in which a melted olefinic polymer composition is extruded through a spinneret 14 to form long fibers 18, and the long fibers 18 are drawn to thinand collected to accumulate on a collecting belt 12 by a suction unit 16 to obtain a spunbond nonwoven fabric 20; a step (II) in which, subsequent to the step (I), a superabsorbent polymer 22 is sprinkled over the spunbond nonwoven fabric 18 on the collecting belt 12; a step (III) in which, subsequent to the step (II), a spunbond nonwoven fabric 34 different from the spunbond nonwoven fabric 18 is layered further on the superabsorbent polymer 22 sprinkled over the spunbond nonwoven fabric 18 on the collecting belt 12 to obtain a laminate; and a step (IV) in which, subsequent to the step (III), a surface shaping processing such as embossing by embossing rolls 36 and 38 is performed on the laminate.

In the step (I), the long fibers 18 extruded through the spinneret 14 may be drawn to reduce the diameter by drawing air (not illustrated).

The step (II) may employ a mode in which the superabsorbent polymer is sprinkled on the spunbond nonwoven fabric intercalating another layer. Alternatively, the step (II) may employ a mode in which the superabsorbent polymer 22 is sprinkled on the spunbond nonwoven fabric 18 together with fluffed synthetic pulp or natural fluff pulp 24. In FIG. 5, the reference numeral 26 denotes a suction unit.

The step (III) may employ a mode in which a spunbond nonwoven fabric 34 different from the spunbond nonwoven fabric 18 is further layered on the superabsorbent polymer 22 sprinkled over the spunbond nonwoven fabric 18, if necessary, intercalating another layer. Such another layer may be a melt-blown nonwoven fabric, or a formed sheet of synthetic pulp.

In the step (III), a spunbond nonwoven fabric 34 may be formed by drawing long fibers 32 extruded through a spinneret 28 to thin, and collecting the long fibers by a suction unit 30 to accumulate on the spunbond nonwoven fabric 18, or the superabsorbent polymer 22 sprinkled over the spunbond nonwoven fabric 18, if necessary intercalating another layer. The absorber is wound up to a roll 40.

<Elastic Nonwoven Fabric>

The absorber according to the invention may further include an elastic nonwoven fabric.

The elastic nonwoven fabric is preferably laminated on the outer side of the absorption layer.

There is no particular restriction on an elastic nonwoven fabric, insofar as it has elasticity to realize a desired body motion followability.

When the absorber includes an elastic nonwoven fabric, body motion followability is imparted, and it is possible to absorb a liquid following a motion of the wearer. Therefore, when the absorber is used, for example, in an adult-use diaper, it may be comfortably used even when going out.

One of preferable modes of an elastic nonwoven fabric is an elastic nonwoven fabric developing elasticity by stretching an elastic nonwoven fabric composed of mixed and spun-bonded combined yarns obtained by extruding simultaneously a polymer containing a thermoplastic polyurethane elastomer, in which the solidification onset temperature in a measurement with a differential scanning calorimeter (DSC) is at least 65C, and the number of particles of polar solvent insoluble matters measured by a particle diameter distribution analyzer based on an electrical sensing zone method equipped with a 100 micron aperture is 3 million/g or less, and a thermoplastic polymer from different nozzles arranged in the same die.

An elastic spunbond nonwoven fabric having the above characteristics may be produced, for example, by the method according to WO 2004/065680.

Another preferable mode of the elastic nonwoven fabric is a method using the elastic nonwoven fabric using a low crystallinity polypropylene according to WO2012 / 070518.

An absorber according to the invention may have another layer in addition to the absorption layer (and an elastic nonwoven fabric to be provided as necessary).

Another layer may be selected according to an application of an absorber according to the invention, and, for example, a layer containing natural fibers may be adopted.

An absorber according to the invention may have two or more absorption layers.

When the absorber according to the invention includes an elastic nonwoven fabric, the absorber according to the invention may include two or more layers of an elastic nonwoven fabric.

Further, when the absorber according to the invention includes another layer, the absorber according to the invention may have two or more other layers.

The thickness of the absorber according to the invention is usually from 0.1 to 3 mm, preferably from 0.1 to 2 mm, and more preferably from 0.1 to 1.5 mm.

The absorption layer of the absorber according to the invention may also be described as a mode constituted with three components from the side in contact with the wearer's skin, an upper, a middle, and a lower component. The upper component preferably contains a hydrophilic crimped fiber having good flexibility and hydrophilicity. The crimped fiber is preferable, because it has good flexibility and retains SAP particles in the fiber layer. Also, owing to good hydrophilicity (especially spot absorbency), it is possible to quickly absorb urine and suppress wearer's skin disorders. The upper component may contain synthetic pulp or natural fluff pulp as mixed. When the synthetic pulp or natural fluff pulp is exposed on the surface, the hydrophilicity (spot absorbency) is improved. Furthermore, since they are branched fibers, the retention performance of SAP is also improved.

The middle component of the absorption layer is preferably a spunbond nonwoven fabric layer or a melt-blown nonwoven fabric layer having a lower hydrophilicity than the upper component.

Here, hydrophilicity is defined by water retention rate. In the absorption layer of an absorber according to the invention, the water retention rate of fibers alone in the upper component is preferably 700% or more, and in the middle component or the lower component the same is preferably less than 700%. In this case, since a body fluid entering the upper component of the absorber diffuses in the lateral directions with respect to the liquid entry direction so that wide-ranging SAP participates in absorption of water to suppress gel blocking.

The lower component of the absorber preferably contains crimped fibers having a high rigidity. When the lower component is composed of crimped fibers having a high rigidity, there is an advantage that a sufficient porosity can be secured and that backflow or leakage of an absorbed body fluid can be suppressed, even when the wearer is sitting or lying and a load is applied.

In this regard, the water retention rate of a nonwoven fabric means herein a value obtained as follows.

Firstly, the weight (Wa (g)) of a nonwoven fabric cut into 100 mm × 100 mm is measured. Next, the nonwoven fabric immersed in pure water for 3 min, then lifted and left in the air for 1 min, and the weight (Wb (g)) is measured again.

The water retention rate of the nonwoven fabric can be calculated by the following equation.

$$\text{Water retention rate of nonwoven fabric (g/g)} = (Wb - Wa)/Wa$$

There is no particular restriction on the application of thw absorber according to the invention, insofar as it is an application for absorbing a liquid, the absorber according to the invention may be used in various applications, such as a sanitary, a medical, or a packaging application.

As preferable applications, cases where the absorber according to the invention is used as an absorber for a sanitary article, such as a diaper, or a sanitary napkin, will be described below.

[Sanitary Article]

A sanitary article of the invention includes the above-described absorber according to the invention.

A sanitary article of the invention is used for a disposable diaper, underpants or a sanitary article, a urine absorbing pad, a sheet for pet, and the like.

Gel blocking to be caused by unbalanced SAP is suppressed in a sanitary article of the invention, even when vibration occurs during transportation, or when the wearer moves around. Consequently, backflow after repeated absorption of a body fluid is suppressed, and the wear's comfort may be maintained even when the article is worn for a long time period.

Examples

The invention will be described in more detail by way of Examples, provided that the invention is not limited to the Examples.

In Examples, the performance of an absorber was evaluated by the following methods.

<Evaluation of Performance of Sanitary Article>

(I) A burette containing 80 mL of physiological saline (0.9 wt% aqueous solution of sodium chloride, the same applies hereinafter) was placed vertically around 10 mm upward from the center of a sanitary article in a size of 475 mm long × 155 mm wide. The time at which the cock was fully opened and the physiological saline began to drop naturally was regarded as "drop start time".

(II) After 180 sec from the drop start time, the area where the physiological saline diffused from the center of a sanitary article was measured, and the value was regarded as the diffusion area of the absorber.

(III) The re-wet amount was measured 480 sec after the drop start time.

More particularly, after 300 sec from the drop start time, 20 sheets of filter paper (ADVANTEC qualitative filter paper No. 2), which mass (Wc (g)) had been previously measured, were stacked at the center of the sanitary article, and a weight of 3.5 kg in a size of 100 mm × 100 mm was placed thereon. After another 180 sec (*i.e.* after 480 sec from the drop start time), the mass (Wd (g)) of the filter paper was measured. The increment of the mass of the filter paper was regarded as the re-wet amount (see the following equation).

$$\text{Re-wet amount (g)} = Wd - Wc$$

The operations from (I) to (III) above were carried out three times in total.

The second drop start time was set at 600 sec after the first drop start time.

The third drop start time was set at 600 sec after the second drop start time.

<Water Absorption Rate of Sanitary Article under Load>

(IV) A 3.5 kg weight in a size of 100 mm $\times$ 100 mm having a cylinder with an inner diameter of 45 mm at the center was placed at the center of an absorber of 475 mm long $\times$ 155 mm wide. In doing so, the weight was so placed that the center of the cylinder of the weight should coincide with the center of the sanitary article.

50 mL of physiological saline was poured from the top of the central cylinder using a measuring cylinder, and the time measurement was started simultaneously, and an elapsed time until the physiological saline disappeared from the surface of the sanitary article was measured. This elapsed time was regarded as the absorption rate.

The measurement of (IV) was carried out three times in total.

Pouring of the physiological saline in the second measurement was performed 600 sec after the disappearance of the physiological saline from the surface of the absorber in the first measurement.

Pouring of the physiological saline in the third measurement was performed 600 sec after the disappearance of the physiological saline from the surface of the absorber in the second measurement.

<Elastic Characteristics>

A test piece of 200 mm (MD) $\times$ 25 mm (CD) was cut out from an absorber and the obtained test piece was stretched by 50% under the conditions of a test piece width of 25 mm, a chuck-to-chuck distance of 30 mm, and a tension rate of 30 mm/min, and then allowed to recover to the current length at the same rate using a universal tensile tester (IM-201 model, manufactured by INTESCO Co., Ltd.). This operation was performed for 2 cycles, and a then generated stress was measured. In a case in which the generated stress was confirmed in the second cycle and a breakage, or the like of the specimen was not recognized, the elastic characteristics was rated as A, and a case in which the conditions were not fulfilled was rated as C.

(Example 1)

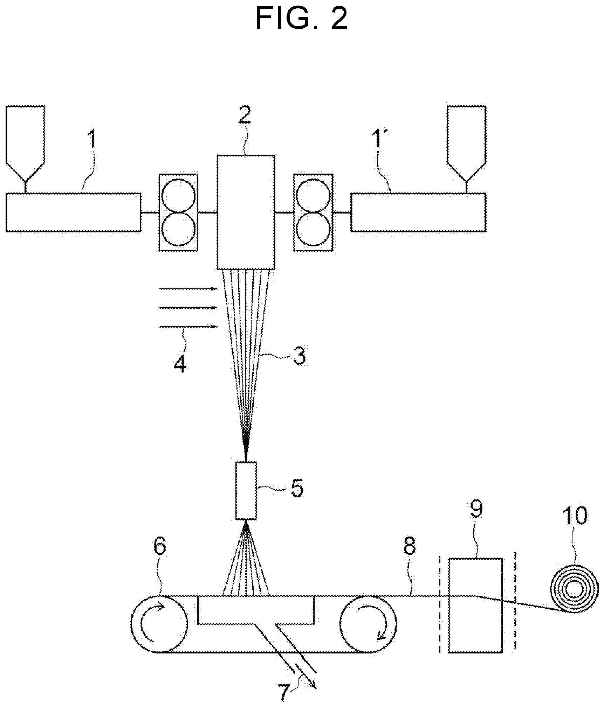

A nonwoven fabric producing apparatus shown in FIG. 2 (spunbonding machine, length in the direction (CD direction) perpendicular to the machine direction (MD direction) on the collecting surface): 300 mm) was made ready for use.

This nonwoven fabric producing apparatus has extruders 1 and 1' for extruding a raw material, a spinneret 2 for spinning the extruded raw material to form fibers 3, an ejector 5 for stretching the fibers 3, a collector device 6 for collecting the stretched fibers to form a nonwoven fabric 8, a bonding unit 9 for thermally bonding at least part of the nonwoven fabric 8, and a winder 10 for winding the nonwoven fabric after thermal bonding. The collector device 6 is equipped with a suction unit 7 for efficiently collecting fibers by suction below a surface for collecting the fibers (collection surface). In this nonwoven fabric producing apparatus, the fibers 3 obtained by the spinneret 2 are cooled by the quench air 4 (air).

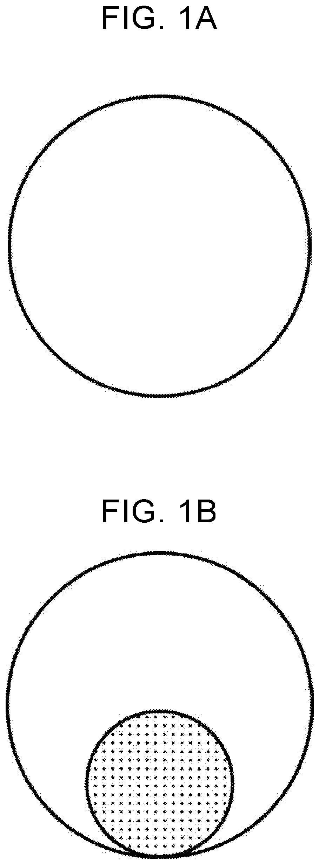

In Example 1, as a spinneret 2, a spinneret for obtaining a fiber having a cross-section shown in FIG. 1A (namely, non-crimped long fiber) was used.

In FIG. 1A, the blank region represents a resin.

A spunbond nonwoven fabric (SB) was produced as follows using the nonwoven fabric producing apparatus.

Using an extruder (75 mm$\phi$), 97 parts by mass of a propylene/ethylene random copolymer having a MFR of 60 g/10 min at 230°C under a load of 2160 g [Mw/Mn = 2.4, melting point (Tm) = 143°C, ethylene content = 4 mol%; hereinafter referred to as "PP-1"], and 3 parts by mass of a glycerin based hydrophilizing agent were melt-blended, and then melt spinning was carried out by a spunbond method, under the condition that the resin temperature and the die temperature were both 200°C, the cooling air temperature was 25°C, and the stretching air flow rate was 2000 m/min, to obtain a non-crimped spunbond nonwoven fabric composed of non-crimped long fibers, having a basis weight of 50 g/m$^2$.

The non-crimped spunbond nonwoven fabric thus obtained was cut into a size of 475 mm long $\times$ 155 mm wide as a non-crimped spunbond nonwoven fabric specimen. Four non-crimped spunbond nonwoven fabric pieces were prepared.

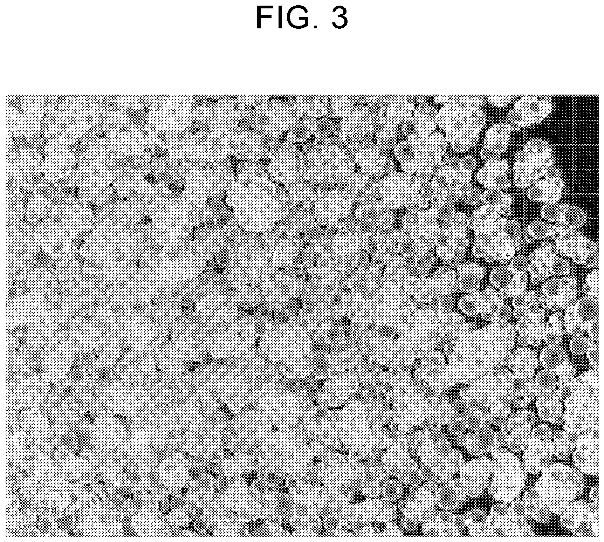

Two sheets of the non-crimped spunbond nonwoven fabric pieces were stacked, and 6.8 g of SAP (sodium polyacrylate crosslinked product, spherical aggregate, particle diameter 350 $\mu$m; an optical micrograph is shown in FIG. 3) was sprinkled thereon evenly.

Two more non-crimped spunbond nonwoven fabric pieces were stacked on the sprinkled SAP.

By conducting hot embossing on the obtained laminate (laminate having the layer structure of SB/SAP/SB), an absorber (absorption layer which was an embossed article) was obtained. The thickness of the absorber thus obtained was as shown in Table 1.

The embossing was conducted using an embossing roll having an emboss pattern shown in FIG. 4 (quilt pattern), an embossing area ratio of 9.7%, and an embossing area (area per emboss pattern) of 3.28 mm$^2$, under conditions that the temperatures of the embossing roll and a smoothing roll were 130C, and the linear pressure was 1 N/mm.

A sanitary article was obtained by bonding a top sheet obtained from a commercially available adult-use diaper to the absorber (absorption layer) after an elapse of 24 hours after the hot embossing.

The results of the performance evaluation are shown in Table 1.

(Example 2)

A nonwoven fabric producing apparatus having the same configuration as the nonwoven fabric producing apparatus used in Example 1 except that the spinneret 2 was changed to a spinneret for obtaining a fiber exhibiting a fiber cross section shown in FIG. 1B (namely, crimped long fiber) was made ready for use.

In FIG. 1B, the blank region and the dotted region represent resins, respectively.

A spunbond nonwoven fabric (SB) was produced as follows using the nonwoven fabric producing apparatus.

In Example 2, as the low melting point thermoplastic resin A, PP-1 (97 parts by mass) and a glycerin based hydrophilizing agent (3 parts by mass) were used, and as the high melting point thermoplastic resin B, a propylene polymer (97 parts by mass) having a MFR of 60 g/10 min at 230C under a load of 2160 g [melting point (Tm) = 157C, hereinafter referred to as "PP-2"] and a glycerin based hydrophilizing agent (3 parts by mass) were used.

The low melting point thermoplastic resin A was melted and blended using an extruder A (75 mm$\phi$), and the high melting point thermoplastic resin B was melted and blended using an extruder B (50 mm$\phi$), and then the two were melt spun by a spunbonding method under the conditions that the resin temperature and die temperature were both 200°C, the cooling air temperature was 25°C, and the stretching air flow rate was 2000 m/min, to obtain a crimped spunbond nonwoven fabric composed of actually crimped conjugate long fibers, having a basis weight of 50 g/m$^2$.

The actually crimped conjugate long fibers of the obtained crimped spunbond nonwoven fabric has a fiber cross section as shown in FIG. 1B. The blank region in FIG. 1B represents a low melting point thermoplastic resin A, and the dotted region in FIG. 1B represents a low melting point thermoplastic resin B.

The crimped spunbond nonwoven fabric thus obtained was cut into a size of 475 mm long $\times$ 155 mm wide as a crimped spunbond nonwoven fabric pieces. Four crimped spunbond nonwoven fabric pieces were prepared.

Two sheets of the crimped spunbond nonwoven fabric pieces were stacked, and 6.8 g of the same SAP as the SAP used in Example 1 was sprinkled thereon evenly.

Two more crimped spunbond nonwoven fabric pieces were stacked on the sprinkled SAP.

By conducting hot embossing under the same conditions as in the hot embossing in Example 1 on the obtained laminate (laminate having the layer structure of SB/SAP/SB), an absorber (absorption layer which was an embossed article) was obtained. The thickness of the absorber thus obtained was as shown in Table 1.

A sanitary article was obtained by bonding a top sheet obtained from a commercially available adult-use diaper to the absorber (absorption layer) after an elapse of 24 hours after the hot embossing.

The results of the performance evaluation of the obtained sanitary article, and the measurement results of the water absorption rate under a load are shown in Tables 1 and 2.

(Example 3)

The same operations as in Example 2 were carried out, except that the SAP amount was changed to 5.1 g, and the measurement of the water absorption rate under a load was omitted.

The results are shown in Table 1.

(Example 4)

The same operations as in Example 2 were carried out, except that the SAP amount was changed to 3.2 g, and the measurement of the water absorption rate under a load was omitted.

The results are shown in Table 1.

(Example 5)

An MB/SB laminate in which a melt-blown nonwoven fabric (MB) was layered on one side of the crimped spunbond nonwoven fabric obtained in Example 2 was produced.

More particularly, a mixture of 93 parts by mass of a propylene homopolymer (MFR = 850 g/10 min, melting point = 159C; hereinafter referred to as "PP-3"), and 7 parts by mass of a glycerin based hydrophilizing agent was supplied to a die of a melt-blown nonwoven fabric producing apparatus. Next, the mixture was extruded from the die set at a temperature of 300°C through a melt blowing nozzle (0.2 mm$\phi$, 30 holes/inch) at an extrusion rate per single nozzle hole of 0.056 g/min together with high temperature and high speed air (300°C, 150 Nm$^3$/hr/m) blown from both the sides of the nozzle onto one side of the crimped spunbond nonwoven fabric to form melt-blown nonwoven fabric on the one side of the crimped spunbond nonwoven fabric to obtain an MB/SB laminate. In doing so, the DCD (the distance from the surface of the spinneret to the collector) was set at 150 mm. The basis weight of the melt-blown nonwoven fabric was set at 5 g/m$^2$.

The crimped spunbond nonwoven fabric obtained in Example 2 was cut into a size of 475 mm long × 155 mm wide as a crimped spunbond nonwoven fabric piece. Three crimped spunbond nonwoven fabric pieces were prepared.

Further, the MB/SB laminate obtained in Example 5 was cut into a size of 475 mm long × 155 mm wide as an MB/SB laminate piece. One MB/SB laminate piece was prepared.

Two sheets of the crimped spunbond nonwoven fabric pieces were stacked, and 6.8 g of the same SAP as the SAP used in Example 1 was sprinkled thereon evenly.

The MB/SB laminate piece was layered on the sprinkled SAP such that the melt-blown nonwoven fabric (MB) was stacked on the SAP side, and one more crimped spunbond nonwoven fabric piece was stacked thereon.

By conducting hot embossing under the same conditions as in the hot embossing in Example 1 on the obtained laminate (laminate having the layer structure of SB/MB/SAP/SB), an absorber (absorption layer which was an embossed article) was obtained. The thickness of the absorber thus obtained was as shown in Table 1.

A sanitary article was obtained by bonding a top sheet obtained from a commercially available adult-use diaper to the absorber (absorption layer) after an elapse of 24 hours after the hot embossing.

The results of the performance evaluation of the obtained sanitary article are shown in Table 1.

(Example 6)

The non-crimped spunbond nonwoven fabric obtained in Example 1 was cut into a size of 475 mm long × 155 mm wide as a non-crimped spunbond nonwoven fabric piece. Four non-crimped spunbond nonwoven fabric pieces were prepared.

Two sheets of the non-crimped spunbond nonwoven fabric pieces were combined and 1 g of synthetic pulp prepared by drying SWP (registered trademark) grade E 505 to a water content of 1% was evenly sprinkled over the range of 350 mm long × 60 mm wide around the center of the non-crimped spunbond nonwoven fabric piece. Further, 6.8 g of the same SAP as used in Example 1 was sprinkled evenly on the area sprinkled with synthetic pulp. On the sprinkled SAP, 2 sheets of the non-crimped spunbond nonwoven fabric pieces were further layered.

By conducting hot embossing under the same conditions as in the hot embossing in Example 1 on the obtained laminate (laminate having the layer structure of SB/SWP/SAP/SB), an absorber (absorption layer which was an embossed article) was obtained. The thickness of the absorber thus obtained was as shown in Table 1.

A sanitary article was obtained by bonding a top sheet obtained from a commercially available adult-use diaper to the absorber (absorption layer) after an elapse of 24 hours after the hot embossing.

The results of the performance evaluation of the obtained sanitary article are shown in Table 1.

(Example 7)

The same spunbond nonwoven fabric as described in Paragraph 0140 of JP-A No. 2015-071854 except that the basis weight of the combined fiber spunbond nonwoven fabric described in Paragraph 0139 of JP-A No. 2015-071854 was changed from 30 g/m$^2$ to 50 g/m$^2$ was cut into a size of 475 mm long × 155 mm wide as an elastic spunbond nonwoven fabric piece. One sheet of the spunbond nonwoven fabric piece was prepared. Further, the crimped spunbond nonwoven fabric obtained in Example 2 was cut into a size of 475 mm long × 155 mm wide as a crimped spunbond nonwoven fabric piece. Four crimped spunbond nonwoven fabric pieces were prepared.

Two sheets of the crimped spunbond nonwoven fabric pieces were stacked on the elastic spunbond nonwoven fabric piece, and further 6.8 g of the same SAP as the SAP used in Example 1 was sprinkled thereon evenly.

Two sheets of the crimped spunbond nonwoven fabric specimens were further stacked on the sprinkled SAP.

By conducting hot embossing under the same conditions as in the hot embossing in Example 1 on the obtained laminate (laminate having the layer structure of elastic SB/SB/SAP/SB), an absorber (absorption layer which was an embossed article) was obtained. The thickness of the absorber thus obtained was as shown in Table 1.

An elastic characteristic measurement was carried out on the obtained absorber (absorption layer) after an elapse of 24 hours after the hot embossing.

The results are shown in Table 1.

(Comparative Example 1)

A core wrap obtained from an adult-use diaper on the market was spread to a size of 475 mm long × 155 mm wide, and then 5.66 g of bleached soft-wood kraft pulp (NBKP) was spread evenly on it.

Over the pulp (NBKP), 6.8 g of the same SAP as the SAP used in Example 1 was sprinkled evenly. On the sprinkled SAP, further 5.66 g of the pulp (NBKP) was layered evenly. Thereafter the core wrap was folded, and appropriate parts are bonded with a spray glue to obtain an absorber.

A sanitary article was obtained by bonding a top sheet obtained from a commercially available adult-use diaper to the absorber (absorption layer).

An elastic characteristic measurement was carried out on the obtained absorber (absorption layer). A performance evaluation and a measurement of water absorption rate with a load were performed on the obtained sanitary article.

The results are shown in Table 1 and Table 2.

[Table 1]

| | | | Example 1 | Example 2 | Example 3 | Example 4 | Example 5 | Example 6 | Example 7 | Comparative Example 1 |
|---|---|---|---|---|---|---|---|---|---|---|
| Structure | | | Non-crimped SB/SAP/SB | Crimped SB/SAP/SB | Crimped SB/SAP/SB | Crimped SB/SAP/SB | Crimped SB/MB/SAP/SB | Non-crimped SB/SWP/SAP/SB | Crimped+Elastic SB/SB/SAP/SB | Core wrap Pulp, SAP |
| SAP amount (g) | | | 6.8 | 6.8 | 5.1 | 3.2 | 6.8 | 6.8 | 6.8 | 6.8 |
| Thickness (mm) | | | 2.0 | 2.2 | 2.2 | 2.2 | 2.3 | 2.5 | 2.7 | 5.5 |
| Absorber Performance evaluation | Diffusion area (mm×mm) | 1st | 287×155 | 269×155 | 264×155 | 282×155 | 325×155 | 250×155 | - | 150×125 |
| | | 2nd | 287×155 | 269×155 | 264×155 | 307×155 | 330×155 | 255×155 | - | 195×130 |
| | | 3rd | 300×155 | 292×155 | 310×155 | 377×155 | 330×155 | 345×155 | - | 210×140 |
| | Re-wet amount (g) | 1st | 0.59 | 0.39 | 0.74 | 3.73 | 0.02 | 0.15 | - | 3.57 |
| | | 2nd | 7.16 | 18.68 | 32.66 | 43.02 | 12.23 | 21.16 | - | 30.84 |
| | | 3rd | 24.60 | 40.70 | 43.81 | 44.65 | 28.29 | 33.72 | - | 42.46 |
| Elastic characteristics | | | - | - | - | - | - | - | A | C |

EP 3 424 475 A1

[Table 2]

| | | Example 1 | Example 2 | Comparative Example 1 |
|---|---|---|---|---|
| Structure | | Non-crimped SB/SAP/SB | Crimped SB/SAP/SB | Core wrap Pulp, SAP |
| SAP amount (g) | | 6.8 | 6.8 | 6.8 |
| Absorption rate under load (sec) | 1st | 39 | 52 | 59 |
| | 2nd | 83 | 47 | 191 |
| | 3rd | 108 | 30 | 280 |

From the results in Table 1, the absorbers of Examples 1 to 6 were wonderfully superior to the absorber of Comparative Example 1 in diffusing capacity. It has been also found that especially with respect to the absorbers of Examples 1, 2, 5, and 6 containing the same amount of SAP as the absorber of Comparative Example 1, the amount of backflow amount (*i.e.* re-wet amount) was smaller, and thus the thickness can be made thinner, compared to Comparative Example 1.

Also, the amounts of backflow in Examples 3 and 4 in which the SAP amounts were reduced were also more or less equal to the amount of backflow in Comparative Example 1.

With respect to each of Examples 1 to 6, after conducting the performance evaluation of the absorber, the interior of the absorber was examined. As a result, it was confirmed that the entire SAP was evenly swollen, and gel blocking phenomenon did not occur.

From the result of Example 7, it was found that by laminating an elastic nonwoven fabric, the absorber acquired elasticity, and thus body motion followability can be provided.

Also, from the results in Table 2, it was found that the absorber of Example 2 was superior in absorption rate under a load to the absorber of Comparative Example 1 or Example 1, and especially superior in the absorption rate from the second time onward.

Considering also the results in Table 1, it was concluded that improvement of the absorption performance by diffusing properly a liquid to utilize a larger amount of SAP has been successfully achieved.

An absorber according to the invention may be applied to absorbent articles in a sanitary material field, an agriculture field, a building material field, and the like. Among others, it may be applied favorably to absorbent articles in a sanitary material field.

The entire contents of the disclosures by Japanese Patent Application No. 2016-042671 are incorporated herein by reference.

All the literature, patent applications, and technical standards cited herein are also herein incorporated to the same extent as provided for specifically and severally with respect to an individual literature, patent applications, and technical standard to the effect that the same should be so incorporated by reference.

**Claims**

1. An absorber having an absorption layer comprising a superabsorbent polymer and a spunbond nonwoven fabric composed of an olefinic polymer composition.

2. The absorber according to claim 1, wherein the olefinic polymer composition comprises a hydrophilizing agent.

3. The absorber according to claim 1 or 2, wherein the spunbond nonwoven fabric is a crimped spunbond nonwoven fabric.

4. The absorber according to any one of claims 1 to 3, wherein the absorption layer is an embossed article.

5. The absorber according to any one of claims 1 to 4, wherein the absorption layer further comprises a melt-blown nonwoven fabric.

6. The absorber according to any one of claims 1 to 5, wherein the absorption layer further comprises a synthetic pulp or a natural fluff pulp.

7. The absorber according to claim 6, wherein the synthetic pulp or the natural fluff pulp is present on a surface of the absorption layer.

8. The absorber according to claim 7, wherein from 0.01 to 10 parts by mass of polypropylene glycol having a molecular weight of from 200 to 10,000 is adhered to 100 parts by mass of the synthetic pulp.

9. The absorber according to any one of claims 1 to 8, further comprising an elastic nonwoven fabric.

10. A sanitary article comprising the absorber according to claim 9.

11. A method of producing an absorber having an absorption layer comprising a superabsorbent polymer and a spunbond nonwoven fabric composed of an olefinic polymer composition, the method comprising:

   step (I): forming long fibers from a melted olefinic polymer composition, drawing to thin the long fibers, and accumulating the long fibers on a collecting belt to obtain a spunbond nonwoven fabric,
   step (II): sprinkling a superabsorbent polymer onto the spunbond nonwoven fabric on the collecting belt,
   step (III): obtaining a laminate by layering, on the collecting belt, a spunbond nonwoven fabric different from the spunbond nonwoven fabric on the collecting belt, and
   step (IV): performing a surface shaping processing on the laminate on the collecting belt.

12. The method of producing an absorber according to claim 11, wherein the olefinic polymer composition comprises a hydrophilizing agent.

13. The method of producing an absorber according to claim 11, wherein the spunbond nonwoven fabric composed of the olefinic polymer composition is a crimped spunbond nonwoven fabric.

14. The method of producing an absorber according to claim 11, wherein in the step (II), the superabsorbent polymer is sprinkled on the spunbond nonwoven fabric composed of the olefinic polymer composition intercalating another layer.

15. The method of producing an absorber according to claim 11, wherein in the step (II), a synthetic pulp or a natural fluff pulp is further sprinkled on the spunbond nonwoven fabric composed of the olefinic polymer composition.

# FIG. 1A

# FIG. 1B

# FIG. 2

# FIG. 3

# FIG. 4

FIG. 5

## INTERNATIONAL SEARCH REPORT

| | International application No. |
|---|---|
| | PCT/JP2017/008613 |

A. CLASSIFICATION OF SUBJECT MATTER
*A61F13/53*(2006.01)i, *A61F13/15*(2006.01)i, *A61F13/534*(2006.01)i

According to International Patent Classification (IPC) or to both national classification and IPC

B. FIELDS SEARCHED

Minimum documentation searched (classification system followed by classification symbols)
A61F13/15-13/84, A61L15/16-15/64

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | JP 2012-105962 A (Kao Corp.),<br>07 June 2012 (07.06.2012),<br>paragraphs [0036], [0048], [0065]; comparative<br>example 5<br>& US 2013/0211358 A1<br>paragraphs [0043], [0055], [0066]; Comparison<br>Example 5<br>& EP 2630939 A1        & CN 103167855 A | 1-2,4-8<br>9-12,14-15<br>3,13 |
| X<br>Y<br>A | WO 2012/043546 A1 (Sumitomo Seika Chemicals<br>Co., Ltd.),<br>05 April 2012 (05.04.2012),<br>paragraphs [0063] to [0069]<br>& US 2013/0178814 A1<br>paragraphs [0088] to [0094]<br>& EP 2623308 A1        & CN 103118867 A | 1-2,4<br>9-12<br>3,5-8,13-15 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>    15 May 2017 (15.05.17) | Date of mailing of the international search report<br>    30 May 2017 (30.05.17) |
|---|---|
| Name and mailing address of the ISA/<br>    Japan Patent Office<br>    3-4-3,Kasumigaseki,Chiyoda-ku,<br>    Tokyo 100-8915,Japan | Authorized officer<br><br><br>Telephone No. |

Form PCT/ISA/210 (second sheet) (January 2015)

**INTERNATIONAL SEARCH REPORT**

International application No.

PCT/JP2017/008613

C (Continuation).    DOCUMENTS CONSIDERED TO BE RELEVANT

| Category* | Citation of document, with indication, where appropriate, of the relevant passages | Relevant to claim No. |
|---|---|---|
| X<br>Y<br>A | JP 2015-100612 A  (Livedo Corp.),<br>04 June 2015 (04.06.2015),<br>claim 3; paragraphs [0015], [0112]; absorbent<br>body 13<br>(Family: none) | 1-4<br>9-13<br>5-8,14-15 |
| Y<br>A | JP 2009-297146 A  (Kao Corp.),<br>24 December 2009 (24.12.2009),<br>paragraph [0028]<br>(Family: none) | 9-10<br>1-8,11-15 |
| Y<br>A | JP 2005-95481 A  (Chisso Corp.),<br>14 April 2005 (14.04.2005),<br>paragraph [0055]; fig. 1<br>(Family: none) | 11-15<br>1-10 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005095481 A
- WO 2010050407 A
- WO 2012129211 A
- WO 2012077638 A
- WO 2011122277 A
- WO 2004065680 A
- WO 2012070518 A
- JP 2015071854 A
- JP 2016042671 A

**Non-patent literature cited in the description**

- Encyclopedia of Chemical Technology. vol. 19, 420-425